# EUROPEAN PATENT SPECIFICATION

(11) **EP 1 696 817 B1**
(45) Date of publication and mention of the grant of the patent: **13.01.2010**
(21) Application number: 04800261.2
(22) Date of filing: 04.11.2004
(51) Int. Cl.: A61C 13/20, B29C 70/06

(54) **ARRANGEMENT AND SYSTEM FOR A DENTAL REPLACEMENT COMPONENT**
ANORDNUNG UND SYSTEM FÜR EINE ZAHNERSATZKOMPONENTE
MECANISME ET SYSTEME DE COMPOSANT DE REMPLACEMENT DENTAIRE

(30) Priority: 10.12.2003 SE 0303310
(43) Date of publication of application: 06.09.2006
(73) Proprietor: Nobel Biocare AB (publ), 402 26 Göteborg (SE)
(72) Inventor: BRAJNOVIC, Izidor, S-416 77 Göteborg (SE)
(74) Representative: Byström, Kurt Linus
(86) International application number: PCT/SE2004/001598
(87) International publication number: WO 2005/055862

(56) References cited:
- SE-B- 457 691
- US-A- 5 829 979
- US-A- 5 846 640
- US-A- 6 132 215

## Description

### FIELD OF INVENTION

The present invention relates, inter alia, to an arrangement for a dental replacement component, which can constitute all or part of a dental bridge, tooth preparation, etc., and which can be given a color corresponding to the area surrounding the replacement component in the patient's mouth (dentine, gums, teeth). The replacement component has a reinforcing element which comprises carbon fiber wires which are arranged in one or more carbon fiber hoses and can be held together by means of hardenable substance so as to form a homogeneous part of the reinforcing element.

The invention also relates to a system for producing a replacement component of this kind.

### BACICGROLJND OF INVENTION

It is already known to use reinforcing elements comprising carbon fiber material, and, on this point, reference may be made to Swedish patent SE 457 691 and to Swedish patent applications 0004883-5, 0203497-3 and 0203897-4.

In the production of a replacement component of the type in question, it is important to be able to provide colors which match the patient's remaining teeth, jaw bone, gums, etc. The use of carbon fiber as reinforcing element gives good results from the point of view of strength. At the same time, however, there is a problem with the carbon fiber's dark color, which has a strong capacity to show through and which is difficult to conceal with opaque covering colors in the form of pink or white.

There is therefore a great need to be able to make the reinforcing element from fibers of another color. The problem is that there is no alternative fiber available on the market with the strength and other physical properties of carbon fiber.

US 6132215 discloses a hybrid fiber-reinforced structural component for dental restorations, wherein the hybrid reinforcing fibers comprise a core having a composite sheath bonded thereto. Preferably, the core comprises a material having a high modulus, such as tungsten, tantalum, niobium, boron or carbon. The composite sheath comprises fibers disposed within a polymeric matrix material, preferably the fibers glass or polyethylene fibers.

### SUMMARY OF INVENTION

The present invention aims to solve this problem and proposes a reinforcing element comprising a core or inner part which is still made of carbon fiber, and a fiber arrangement which lies on the outside of this and is more suitable than the carbon fiber in terms of its color.

In an aspect, there is provided an arrangement for a dental replacement component, which can be given a coloring which matches the area surrounding the replacement component, the replacement component having a reinforcing element which comprises carbon fiber wires, which are arranged in one or more carbon fiber hoses and can be held together by means of hardenable substance so as to form a homogeneous part of the reinforcing element, wherein the carbon fiber part, at least in its portion or portions directed toward the surrounding area, supports or is provided with additional fiber material with a color which better matches said coloring than does the carbon fiber (8), **characterized in that** said additional fiber material comprises or consists of aluminum oxide fibers or para-aramid fibers.

In embodiments when the additional fiber material comprises or consists of aluminum oxide fibers, the additional fiber material may be substantially white. In embodiments when the additional fiber material comprises or consists of para-aramid fibers, the additional fiber material may be substantially yellow.

In an embodiment, the carbon fiber part, at least in its portion or portions directed toward the surrounding area, may support or may be provided with additional fiber material with a color which better matches said coloring than does the carbon fiber.

The additional fiber material can have a tube shape and can enclose all or said portion/portions of the carbon fiber. Besides covering the dark color of the carbon fiber, the additional fiber material can, if so desired, also increase the stiffness and/or strength of the homogeneous carbon fiber part.

In another embodiment, the layer or thickness of the additional fiber material will be 2-5% of the thickness or diameter of the reinforcing element. Further embodiments of the novel arrangement are set out in the attached dependent claims relating to the arrangement.

In another aspect, there is provided a system for producing a dental replacement component, which can be given a coloring which matches the area surrounding the component, said component having a reinforcing element which comprises carbon fiber wires which are arranged in one or more carbon fiber hoses and can be held together by means of hardenable substance so as to form a homogeneous part of the reinforcing element, **characterized in that** identification equipment is designed to identify the treatment situation on the patient, and to transmit information to computer equipment which, as a function of said information and through interaction with a user, is intended to permit display, on screen, of the replacement component and of a reinforcing element included in said component and having a core or inner part of carbon fiber, and fibers completely or partially covering the core or inner part and made of a material, which comprises or consists of aluminum oxide or para-aramid, having a color which better matches said coloring than does the carbon fiber, and in that equipment involving considerable manual input and/or substantially fully automated equipment is arranged to take part in production of the reinforcing element on the basis of empirical data concerning the core and the fiber covering this, for example with the aid of data on diameters, thicknesses, qualities, color, relations between the carbon fiber and the additional fiber, etc.

By means of what has been proposed above, it is possible to obtain what could be regarded as a hybrid. The carbon fiber is used as reinforcement for production of dental bridges on implants or prepared teeth. The dark color of the carbon fiber is not easy to change, which generally is a considerable disadvantage in the production of dental bridges. The fiber therefore has to be painted with a covering color (pink or white) depending on whether the aim is to conceal it against the gum or the teeth. A desire to be able to use a white fiber has therefore been expressed. The aluminum oxide fiber is white in color and is therefore widely accepted in this application. By means of what is proposed, a number of carbon fiber hoses can be fitted inside a hose made of aluminum oxide, with the result that a hybrid is obtained which solves the problem concerning the dark color of the carbon fiber. The core of carbon fiber can be responsible for providing the good physical properties, and the aluminum oxide fiber covers the dark color of the carbon fiber and increases the stiffness of the construction, when so desired. Another fiber which can be used correspondingly is the para-aramid fiber sold under the trade name KEVLAR or TWARON®. This fiber is more yellow in color, which is preferable to the dark color of the carbon fiber. Said para-aramid fibers can be used if one accepts moisture absorption of up to ca. 3%. If lower moisture absorption levels, for example 0.1%, are required, aluminum oxide fibers can be used.

### BRIEF DESCRIPTION OF THE DRAWINGS

A presently proposed embodiment of an arrangement and a system according to the invention will be described below with reference to the attached drawings, in which
- Figure 1: shows a front view of a replacement component in the form of a dental bridge fitted to an upper jaw and provided with a reinforcing element, and showing its set of teeth in relation to the teeth of a lower jaw,
- Figure 2: shows a top view of the extent of the reinforcing element in the replacement component according to Figure 1,
- Figure 3: shows a cross section through a reinforcing element consisting of an inner core of carbon fiber and a tube surrounding the core and made of a second fiber material,
- Figure 4: shows the reinforcing element according to Figure 3 in longitudinal section,
- Figure 5: shows a cross section through an inner layer of carbon fiber material, and a part made of a second fiber material partially covering the inner layer,
- Figure 6: shows a front view of a replacement component comprising a tooth preparation with reinforcing element, and
- Figure 7: is a flow chart showing the various steps in the production of a replacement component.

### DETAILED DESCRIPTION OF EMBODIMENTS

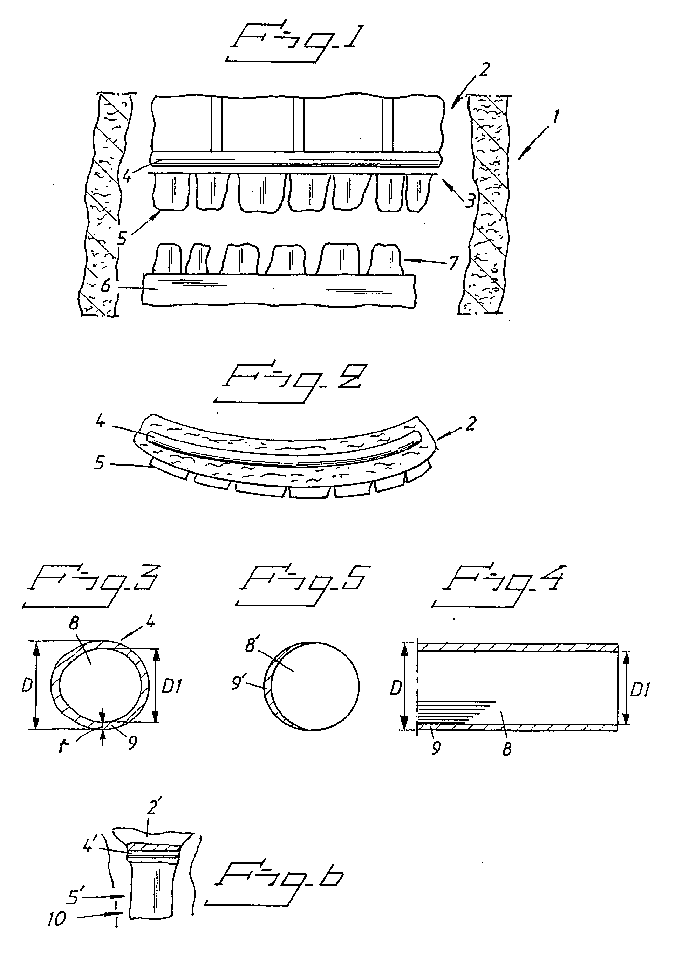

In Figure 1, a patient is indicated symbolically by 1. The patient's upper jaw 2 is provided with a replacement component in the form of a dental bridge 3. The replacement component can be designed in a manner known per se with internal reinforcing element 4 and set of teeth 5. In Figure 1, the patient's lower jaw is also indicated symbolically by 6 and the patient still has teeth 7 in the lower jaw.

Figure 2 shows the extent of the reinforcing element 4 in the upper jaw 2. The teeth are likewise indicated symbolically by 5.

Figure 3 shows that the reinforcing element 4 comprises a core 8 of carbon fiber and a tube 9 which surrounds the core and is made of a second fiber material which may be aluminum oxide or para-aramid. The diameter of the reinforcing element is indicated by D, and the diameter of the core is indicated by D1. The additional fiber layer 9 has a thickness t which is substantially less than the diameter D1. The thickness t can, for example, be 1-5% of the diameter D1.

The carbon.fiber can, in a manner known per se, be made up of carbon fiber wires inlaid from the outset in carbon fiber hoses. The additional fiber 9 thus forms a hose for the carbon fiber wires or carbon fiber hoses surrounding these. The surrounding hose 9 has been provided with hardening agent which has the effect that the carbon fiber wires and the carbon fiber hoses are held firmly together in connection with polymerization. The formation of a homogeneous carbon fiber core can be effected in accordance with the literature references mentioned in the introduction and will therefore not be described in any detail here. Instead of a described tube which is removed after the polymerization, use is thus made of the additional fiber material, which is therefore retained on the finished reinforcing element.

Figure 5 shows an illustrative embodiment in which the core 8' is only partially covered by the additional fiber material 9'. In connection with this embodiment, it is possible to turn that part of the reinforcing element covered with the fiber material 9' to face the outside of the replacement component.

Figure 6 shows a tooth preparation which can use a reinforcing element according to the invention. In this case, the tooth preparation comprises only one tooth and is indicated symbolically by 10. The patient's upper jaw is indicated symbolically by 2', and the reinforcing element by 4', while the tooth in question is indicated symbolically by 5'.

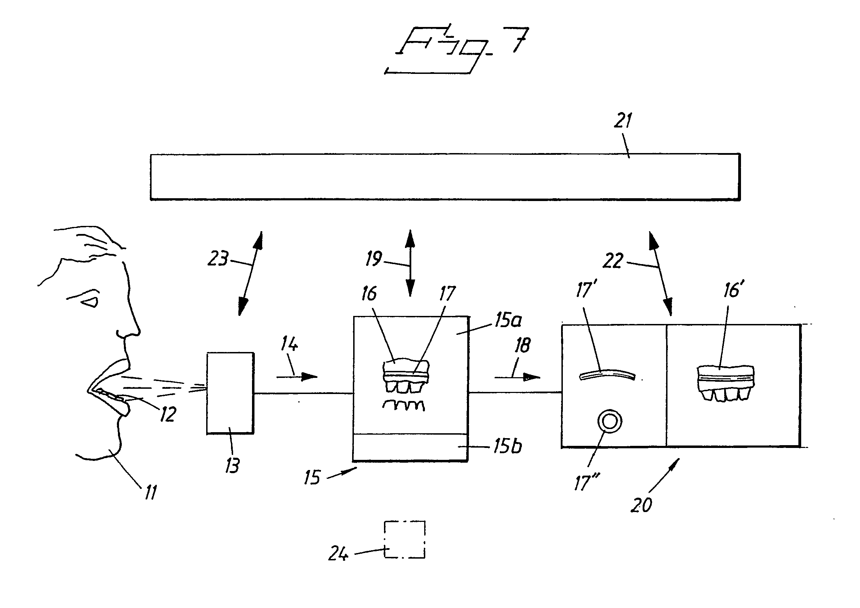

In Figure 7, the patient is indicated by 11. The patient is to receive a dental bridge in the upper jaw and still has teeth 12 in his lower jaw. Identification equipment is indicated by 13 and, with the aid of this identification equipment, the treatment situation on the patient can be identified. This identification can be done in a manner known per se using different equipment, instruments and aids. Information 14 relating to the result of the identification can be transmitted to computer equipment 15 which is provided with a screen 15a and operating member (e.g. keyboard) 15b. The computer equipment is intended, in a manner known per se, to provide a visual image of the treatment situation as a function of the received information 14 and to add modifications to this image via the operating member 15b. In this way, a visual image 16 of the replacement component/dental bridge can be obtained. The fitting of the reinforcing element is indicated symbolically by 17 in Figure 7. As a function of the visual structure on the computer 15, information 18, 19 concerning this can be transmitted to manually operated equipment 20 (at a dental technician) and/or to fully automated production equipment for dental products, for example production equipment of the PROCERA® type. If the information from the computer equipment is received in the equipment 20, the reinforcing element 17', 17" can be produced and the dental bridge or replacement component 16' finished. Correspondingly, said production of reinforcing element and dental bridge can be carried out in the fully automated equipment 21. Alternatively, cooperation is also possible between the units 20 and 21, as has been symbolized by arrow 22 pointing in two directions. The equipment 21 can also be in two-way communication with the computer equipment, see 19, and the identification equipment, see arrows 23. In connection with the production in the equipment 20 and/or equipment 21, the replacement component can be produced on the basis of empirical data. In this respect it may be mentioned that information can be obtained concerning the core and the fiber covering it. Such information can include details of diameters, thicknesses, qualities, colors, relations between diameters and thicknesses for the situation in question. The color chosen for the additional fiber material can be white (or substantially white) or can have a yellow shade. Information on the moisture absorption to be present in the particular case of treatment can also be received. In Figure 7, a user of the computer equipment is indicated symbolically by 24.

The computer equipment and identification equipment, which can be of known types, operate with conventional computer programs and file management.

## Claims

1. An arrangement for a dental replacement component, which can be given a coloring which matches the area surrounding the replacement component , the replacement component having a reinforcing element (4) which comprises carbon fiber wires, which are arranged in one or more carbon fiber hoses and can be held together by means of hardenable substance so as to form a homogeneous part of the reinforcing element, wherein the carbon fiber part, at least in its portion or portions directed toward the surrounding area, supports or is provided with additional fiber material with a color which better matches said coloring than does the carbon fiber (8), **characterized in that** said additional fiber material comprises or consists of aluminum oxide fibers or para-aramid fibers.

2. The arrangement as claimed in patent claim 1, **characterized in that** the additional fiber material has a tube shape and encloses all or said portion/portions of the carbon fiber.

3. The arrangement as claimed in any of patent claims 1-2, **characterized in that** the additional fiber material is provided with moisture absorption corresponding to or only slightly exceeding the moisture absorption of the carbon fiber.

4. The arrangement as claimed in any of patent claims 1-3, **characterized in that** the layer or wall thickness of the additional fiber material is 1-5% of the thickness or diameter of the reinforcing element.

5. The arrangement as claimed in any of patent claims 1-4, **characterized in that** carbon fiber hoses with carbon fiber wires are placed in a hose made of the additional fiber material.

6. A system for producing a dental replacement component, which can be given a coloring which matches the area surrounding the component, said component having a reinforcing element (4) which comprises carbon fiber wires which are arranged in one or more carbon fiber hoses and can be held together by means of hardenable substance so as to form a homogeneous part of the reinforcing element, **characterized in that** identification equipment (13) is designed to identify the treatment situation on the patient and to transmit information to computer equipment (15) which, as a function of said information and through interaction with a user (24), is intended to permit display, on screen, of the replacement component and of a reinforcing element included in said component and having a core (8) or inner part of carbon fiber, and fibers (9, 9') completely or partially covering the core or inner part and made of a material, which comprises or consists of aluminum oxide or para-aramid, having a color which better matches said coloring than does the carbon fiber, and **in that** equipment involving considerable manual input and/or substantially fully automated equipment is arranged to take part in production of the reinforcing element on the basis of empirical data concerning the core and the fiber covering this, for example with the aid of data on diameters (D, D1), thicknesses (t), qualities, color, relations between the carbon fiber and the additional fiber, etc.

## Patentansprüche

1. Anordnung für eine Zahnersatzkomponente, der eine Färbung verliehen werden kann, die mit dem die Ersatzkomponente umgebenden Bereich übereinstimmt, wobei die Ersatzkomponente ein Verstärkungselement (4) aufweist, das Kohlefaserdrähte umfasst, die in einem oder mehreren Kohlefaserschläuchen angeordnet sind und mittels einer härtbaren Substanz zusammen gehalten werden können, um einen homogenen Teil des Verstärkungselements zu bilden, wobei der Kohlefaserteil wenigstens an seinem Teil oder Teilen, die zum umgebenden Bereich weisen, ein zusätzliches Fasermaterial trägt oder mit diesem versehen ist, das eine Farbe aufweist, die besser mit der Färbung übereinstimmt als die Kohlefaser (8), **dadurch gekennzeichnet, dass** das zusätzliche Fasermaterial aus Aluminiumoxidfasern oder para-Aramidfasern besteht oder diese aufweist.

2. Anordnung nach Patentanspruch 1, **dadurch gekennzeichnet, dass** das zusätzliche Fasermaterial eine Rohrform hat und alle oder den Teil/die Teile der Kohlefasern umschließt.

3. Anordnung nach einem der Patentansprüche 1 und 2, **dadurch gekennzeichnet, dass** das zusätzliche Fasermaterial mit einer Feuchtigkeitsaufnahme versehen ist, die der Feuchtigkeitsaufnahme der Kohlefasern entspricht oder diese nur geringfügig überschreitet.

4. Anordnung nach einem der Patentansprüche 1-3, **dadurch gekennzeichnet, dass** die Schicht- oder Wanddicke des zusätzlichen Fasermaterials 1-5% der Dicke oder des Durchmessers des Verstärkungselementes ist.

5. Anordnung nach einem der Patentansprüche 1-4, **dadurch gekennzeichnet, dass** Kohlefaserschläuche mit Kohlefaserdrähten in einem Schlauch, hergestellt aus dem zusätzlichen Fasermaterial, platziert sind.

6. System zur Herstellung einer Zahnersatzkomponente, der eine Färbung verliehen werden kann, die mit dem die Komponente umgebenden Bereich übereinstimmt, wobei die Komponente ein Verstärkungselement (4) aufweist, das Kohlefaserdrähte aufweist, die in einem oder mehreren Kohlefaserschläuchen angeordnet sind und mittels einer härtbaren Substanz zusammen gehalten werden können, um einen homogenen Teil des Verstärkungselementes zu bilden, **dadurch gekennzeichnet, dass** eine Identifikationsausrüstung (13) so gestaltet ist, dass sie die Behandlungssituation am Patienten identifizieren kann und Informationen auf eine Computerausrüstung (15) übertragen kann, die, als eine Funktion dieser Information und durch Interaktion mit einem Benutzer (24), dafür gedacht ist, eine Anzeige, auf einem Bildschirm anzuzeigen, der Zahnersatzkomponente und eines Verstärkungselementes, das in der Komponente enthalten ist, und einen Kern (8) oder einen inneren Teil aus Kohlefaser hat, und Fasern (9,9'), die den Kern oder inneren Teil vollständig oder teilweise abdecken und aus einem Material bestehen, das aus Aluminiumoxid oder para-Amid besteht oder dieses aufweist, mit einer Farbe, die besser mit der Färbung als die Kohlefaser übereinstimmt, und dass die Ausrüstung eine beträchtliche manuelle Eingabe umfasst und/oder eine im Wesentlichen vollständig automatische Ausrüstung angeordnet ist, um an der Herstellung des Verstärkungselementes auf der Basis empirischer Daten bezüglich des Kerns und der diesen abdeckenden Fasern teilzuhaben, beispielsweise mit Hilfe von Daten bezüglich des Durchmessers (D, D1), der Dicken (t), der Qualitäten, Farbe, Beziehungen zwischen der Kohlefaser und der zusätzlichen Faser etc.

## Revendications

1. Mécanisme de composant de remplacement dentaire, auquel peut être attribuée une coloration qui s'accorde avec la zone entourant le composant de remplacement, le composant de remplacement ayant un élément de renfort (4) qui comprend des fils en fibre de carbone, qui sont agencés en un ou plusieurs tubes flexibles en fibre de carbone et qui peuvent être maintenus ensemble au moyen d'une substance durcissable de sorte à former une partie homogène de l'élément de renfort, dans lequel la partie en fibre de carbone, au moins dans sa portion ou ses portions dirigée(s) vers la zone environnante, supporte ou est dotée d'un matériau de fibre supplémentaire ayant une couleur qui s'accorde mieux à ladite coloration que ne le fait la fibre de carbone (8), **caractérisé en ce que** ledit matériau de fibre supplémentaire comprend ou est constitué de fibres d'oxyde d'aluminium ou de fibres para-aramides.

2. Mécanisme selon la revendication 1, **caractérisé en ce que** le matériau de fibre supplémentaire a une forme de tube et enferme l'ensemble ou ladite/lesdites portion(s) de la fibre de carbone.

3. Mécanisme selon l'une quelconque des revendications 1 et 2, **caractérisé en ce que** le matériau de fibre supplémentaire présente une absorption d'humidité correspondant à l'absorption de l'humidité de la fibre de carbone ou la dépassant légèrement.

4. Mécanisme selon l'une quelconque des revendications 1 à 3, **caractérisé en ce que** l'épaisseur de couche ou de paroi du matériau de fibre supplémentaire représente 1 à 5 % de l'épaisseur ou du diamètre de l'élément de renfort.

5. Mécanisme selon l'une quelconque des revendications 1 à 4, **caractérisé en ce que** les tubes flexibles en fibre de carbone avec les fils en fibre de carbone sont placés dans un tube flexible constitué du matériau de fibre supplémentaire.

6. Système pour produire un composant de remplacement dentaire, auquel peut être attribuée une couleur qui s'accorde à la zone entourant le composant, ledit composant ayant un élément de renfort (4) qui comprend des fils en fibre de carbone qui sont agencés en un ou plusieurs tubes flexibles en fibre de carbone et qui peuvent être maintenus ensemble au moyen d'une substance durcissable de sorte à former une partie homogène de l'élément de renfort, **caractérisé en ce que** l'équipement d'identification (13) est conçu pour identifier la situation de traitement sur le patient et pour transmettre les informations à l'équipement informatique (15) qui, en fonction desdites informations et via une interaction avec un utilisateur (15), est sensé permettre l'affichage, sur l'écran, du composant de remplacement et d'un élément de renfort inclus dans ledit composant et ayant un coeur (8) ou une partie interne de fibre de carbone, et des fibres (9, 9') recouvrant complètement ou partiellement le coeur ou la partie interne et constituées d'un matériau, qui comprend ou est constitué d'oxyde d'aluminium ou para-aramide, ayant une couleur qui s'accorde mieux à ladite coloration que ne le fait la fibre de carbone, et **caractérisé en ce qu'**un équipement impliquant une entrée manuelle considérable et/ou un équipement complètement automatisé est agencé pour prendre part à la production de l'élément de renfort sur la base de données empiriques concernant le coeur et la fibre le recouvrant, par exemple, à l'aide de données sur les diamètres (D, D1), les épaisseurs (t), les qualités, la couleur, les relations entre la fibre de carbone et la fibre supplémentaire, etc.
